(19) Europäisches Patentamt
European Patent Office
Office européen des brevets

(11) EP 4 588 428 A1

(12) EUROPEAN PATENT APPLICATION

(43) Date of publication:
23.07.2025 Bulletin 2025/30

(51) International Patent Classification (IPC):
A61B 5/00 (2006.01)    A61B 5/107 (2006.01)

(21) Application number: 24152650.8

(22) Date of filing: 18.01.2024

(52) Cooperative Patent Classification (CPC):
A61B 5/1079; A61B 5/0075; A61B 5/1075;
A61B 5/489

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(71) Applicant: Nederlandse Organisatie voor
toegepast-natuurwetenschappelijk Onderzoek
TNO
2595 DA 's-Gravenhage (NL)

(72) Inventors:
• AMELINK, Arjen
  2595 DA 's-Gravenhage (NL)
• STUART-WIJESURIYA, Shavini Ann Ediriweera
  2595 DA 's-Gravenhage (NL)
• ZOUTENBIER, Vincent Sean
  2595 DA 's-Gravenhage (NL)

(74) Representative: V.O.
P.O. Box 87930
2508 DH Den Haag (NL)

(54) METHOD AND SYSTEM FOR DETERMINING A BLOOD VESSEL CALIBER BELOW THE SKIN

(57) A method and system for determining blood vessel caliber (Dv) of blood vessels (B) below a surface of skin (S). The skin (S) is illuminated with source light having at least a first wavelength (Xa) and a second wavelength ($\lambda$b). The source light is configured to penetrate into the skin (S) for interacting with the blood vessels (B) below the skin (S). A first time-dependent signal (Sa) is measured to indicate an intensity variation (Ia[t]) of light at the first wavelength ($\lambda$a) emanating from the skin (S), resulting from the respective source light after having interacted with the blood vessels below the skin. A second time-dependent signal (Sb) is measured to indicate an intensity variation (Ib[t]) of light at the second wavelength ($\lambda$b). An indication of the blood vessel caliber (Dv) is determined based on a combination of the first time-dependent signal (Sa) and the second time-dependent signal (Sb).

FIG 1

**Description**

TECHNICAL FIELD AND BACKGROUND

The present disclosure relates to a method and a system for measuring an indication of a blood vessel caliber of blood vessels below a skin tissue.

Blood vessel health is crucial for diagnosing and predicting risk for diseases like heart attacks, strokes, or aneurysms, and in evaluating the efficacy of medications. Globally, cardiovascular diseases are the leading cause of death. Traditionally, monitoring blood vessel diameters has been confined to medical facilities like hospitals, offering only snapshot assessments and limiting the ability to diagnose conditions or assess drug efficacy accurately.

Current methods, such as ultrasonic equipment or intravital microscopy, require substantial hardware, impeding daily life and unsuitable for long-term monitoring. In microcirculation studies, blood vessel diameters are measured to assess the impact of various stimuli, typically using intravital video microscopy. This process involves recording lengthy video sequences, resulting in thousands of images, and requires significant hardware, which is typical in the medical industry. Consequently, devices like echocardiograms, cardiovascular cartography (CCG), or electroencephalography (EEG) are often too cumbersome for regular use.

Recently, there has been a shift towards medical wearables, making patient monitoring at home feasible over extended periods. Currently, commercial wearables are available for monitoring heart or brain functions. However, existing methods and devices primarily focus on hemoglobin oxygen saturation in blood flow, not directly on measuring blood vessel calibers. For example, photoplethysmography (PPG) involves calculating time-invariant or offset signals and time-varying components to estimate oxygen saturation and heart rate, as shown in US2002042558 A1. Similarly, EP3745957 A1 describes methods for measuring retinal oxygenation, including pigment packaging effects (PPE).

There is a need for improved methods and systems which can robustly measure blood vessel caliber without relying on complicated hardware or visits to medical facilities. The development of such a method would represent a significant advancement in patient monitoring and diagnostics.

SUMMARY

Aspects relate to methods and systems method for determining blood vessel caliber of blood vessels below the skin. By employing source light with multiple wavelengths, including at least a first, second, and preferably a third wavelength, the light may effectively penetrate the skin to interact with subdermal blood vessels. Each wavelength can be chosen for its unique interaction with the blood vessels, e.g. characterized by distinct absorption coefficients and/or varying absorption dependencies on the vessel caliber. The inventor finds that, by measuring time-dependent signals corresponding to two or more of these wavelengths, where each signal represents the intensity variation of light emanating from the skin after interacting with the blood vessels, a more accurate indication of blood vessel caliber may be determined. For example, this indication can be derived from a combination of at least a first and a second time-dependent signal, leveraging the differential properties of the wavelengths to enhance the precision of the vessel caliber assessment.

By measuring the time-dependent signals simultaneously, more accurate and consistent assessment of blood vessel caliber may be determined, e.g. by ensuring that the data reflects the same physiological state of the blood vessels, reducing potential discrepancies. By extracting overall time-varying and offset components from each of the respective time-dependent signals, the indication of blood vessel caliber may be more easily or accurately determined using these components. For example, the overall components may be less affected by random noise and motion/movement artefacts. In one embodiment, an offset component may be determined by calculating an average or median value from a plurality of measurement points in the respective time-dependent signal. In some embodiments, the offset component may also be split into a fixed offset (e.g. due to overall loss of light between the light source and detector), and a wavelength dependent offset (e.g. attributable to actual absorption in the blood vessels). In another or further embodiment, a time-varying component may be determined by calculating a standard deviation, variance, root mean square, peak-to-peak amplitude, min-max, or value quantifying an amplitude of time-dependent variation in the respective signal. The inventor finds that, the blood vessel caliber can be assessed particular well using the ratio of the time-varying components to their respective offset components. For example, this reduces systematic errors and noise that are particularly prominent in long-term measurements on moving subjects.

By ensuring that a significant portion of the measured light (e.g., over 50%) at the first and second wavelengths has a penetration depth in the range of one to five millimeters, preferably between one and three millimeters, superficial blood vessels below the skin may be particularly targeted. The inventor finds that this specific depth range can achieve the most precise indication for the blood vessel caliber of the microcirculation. For example, the blood vessels at this shallow depth may be relatively small and therefore most affected by any changes, as well as being more affected by the so-called 'pigment packaging effect', described herein.

The inventor finds that, by choosing 'blue' light in the 400 - 450 nm range and 'green' light in the 500 - 600 nm range as the first and second wavelengths, respectively, the method may further enhance microcirculatory blood vessel caliber measurement. While the blue light, especially in the 400 - 450 nm range, is significantly affected by the pigment packaging parameter, providing reliable insights for caliber determination, the green light in the 500 - 600 nm range is less influenced by this effect, while still probing the same depth range as the blue light (i.e. interrogating the same superficial microvessels). This lesser impact on green light results in a different interaction with the blood vessels. The combined analysis of these two wavelengths, each with their distinct dependencies, allows for a more comprehensive and accurate determination of blood vessel caliber. The differential responses of blue and green light to pigment packaging and vessel characteristics may thus improve the overall precision of the measurement.

In some embodiments, a third wavelength, distinct from both the first and second wavelengths is used. This third wavelength, ideally 'red' light selected in the range of 600 - 900 nm, may result in the measurement of a third time-dependent signal. Advantageously, the 'red' light selected in said wavelength range may be substantially independent of the blood vessel caliber, or at least less dependent on it compared to the 'blue' and 'green' wavelengths. The inventor finds that relative independence can make the 'red' light particularly advantageous for obtaining additional information about the offset component present in the signal. By combining the 'red' wavelength in with at least one of the 'blue' wavelength and 'green' wavelength, preferably both, more comprehensive data may be gathered. The inventor finds that using three distinct wavelengths, each with its unique interaction properties, may further enhance the overall accuracy and reliability of the blood vessel caliber assessment.

As demonstrated herein, the light at different wavelengths may have different penetration depths below the skin, which could potentially lead to the different time-dependent signals measuring different vessels. This variation may pose a challenge in accurately assessing blood vessel caliber consistently across multiple wavelengths. This effect is mitigated by combining blue and green wavelengths, for which the penetration depths are similar. To further alleviate this issue, a possible solution involves adjusting the measurement distances between the respective light source and sensor for each wavelength. Specifically, a closer distance can be used to compensate for the light with a higher penetration depth. By doing so, the system effectively standardizes the depth at which blood vessels are measured by each wavelength, ensuring that the same vessels are assessed despite the inherent differences in light penetration. This strategy of varying measurement distances may thus improve consistency and reliability of the blood vessel caliber determination across different wavelengths, especially for simultaneously measured time-dependent signals.

In some embodiments, the blood vessel caliber may be determined based on an analytical model which includes an effective absorption coefficient of the blood vessels at the one or more wavelengths. The analytical model may be calculated as function of predetermined absorption coefficients of homogeneously distributed oxy- and deoxyhemoglobin at the one or more wavelengths weighted according to an oxygenation defined as a fraction of one of the oxy- and deoxyhemoglobin over a total hemoglobin. The analytical model further comprises a pigment packaging factor. The pigment packaging factor depending on a caliber of the blood vessel to determine a pigment packaging effect of absorbing hemoglobin molecules not being homogeneously distributed throughout the blood vessel but contained in discrete package of the blood vessel. The blood vessel caliber being calculated based on the pigment packaging effect. The pigment packaging effect may give more robust indication of a caliber of a blood vessel.

In will be understood that aspects of the present disclosure may also be embodied as program instructions and/or a non-transitory computer-readable medium storing instructions that, when executed by one or more processors, cause a device to perform one or more of the methods described herein. Other or further aspects may also be embodied in suitable hardware, e.g. one or more light sources configured to illuminate the skin with source light having respective wavelengths; one or more light sensors configured to measure respective time-dependent signals; a processor configured to determine the indication of the blood vessel caliber based on a combination of the time-dependent signals; and an optional display or other output device for showing the blood vessel caliber, as determined by the system.

BRIEF DESCRIPTION OF DRAWINGS

These and other features, aspects, and advantages of the apparatus, systems and methods of the present disclosure will become better understood from the following description, appended claims, and accompanying drawing wherein:

FIG 1 displays a device configured to measure the blood vessel caliber of blood vessels below the skin;
FIG 2A displays an expanded view of the source light depicted in FIG 1;
FIG 2B displays an expanded view of the detector depicted in FIG 1;
FIG 3A displays a result of absorption coefficient in function of wavelength for different blood vessel calibers;
FIG 3B displays an amplitude-graph as time dependent signal for the first wavelength;
FIG 3C displays an amplitude-graph as time dependent signal for the second wavelength;
FIG 4 displays various wavelengths and their penetration depth below the skin.

DESCRIPTION OF EMBODIMENTS

Terminology used for describing particular embodiments is not intended to be limiting of the invention. As used herein, the singular forms "a", "an" and "the" are intended to include the plural forms as well, unless the context clearly indicates otherwise. The term "and/or" includes any and all combinations of one or more of the associated listed items. It will be understood that the terms "comprises" and/or "comprising" specify the presence of stated features but do not preclude the presence or addition of one or more other features. It will be further understood that when a particular step of a method is referred to as subsequent to another step, it can directly follow said other step or one or more intermediate steps may be carried out before carrying out the particular step, unless specified otherwise. Likewise it will be understood that when a connection between structures or components is described, this connection may be established directly or through intermediate structures or components unless specified otherwise.

As used herein, the term "blood vessel caliber", refers to the diameter of most blood vessels in cardiovascular and peripheral vascular system. As used herein, the term "DC component", refers to a quasi-static component corresponding to light diffusion through tissues and non-pulsatile blood layers. As used herein, the term "fixed offset DC component", refers to the quasi static component corresponding to light diffusion related to electrical noise. As used herein, the term "actual offset DC component", refers to quasi static component corresponding to light diffusion through tissues, non-pulsatile blood layers. As used herein, the term " AC component", refers to a time dependent component corresponding to light diffusion through pulsatile blood layers or vessels.

The invention is described more fully hereinafter with reference to the accompanying drawings, in which embodiments of the invention are shown. In the drawings, the absolute and relative sizes of systems, components, layers, and regions may be exaggerated for clarity. Embodiments may be described with reference to schematic and/or cross-section illustrations of possibly idealized embodiments and intermediate structures of the invention. In the description and drawings, like numbers refer to like elements throughout. Relative terms as well as derivatives thereof should be construed to refer to the orientation as then described or as shown in the drawing under discussion. These relative terms are for convenience of description and do not require that the system be constructed or operated in a particular orientation unless stated otherwise.

With population aging, more and more pressure will be set in the public health system, increasing expenditure for governments. Further, at present the efficacy of medications is rather difficult to predict and lengthy trials will need to be done. Furthermore, measurements of microvascular constriction and dilation may provide early understanding in response to food allergens. An early exposure to food allergens would result in an early vasodilation or vasoconstriction event. Additionally, an effective way of predicting the efficacy of medication, e.g. cardiovascular medication, would be to measure the caliber (constriction and dilation) of a blood vessel. Moreover, cardiovascular diseases are known to be the number 1 cause of death. Early insights into vascular diseases would possibly reduce the number of casualties. One possible way to receive early insights is to monitor calibers of multiple blood vessels for an extended period of time.

Constriction and dilation of a blood vessel is often referred to as vasotonia. Vasotonia refers to the state of tone or tension in the walls of blood vessels. It essentially signifies the level of contraction or relaxation of the smooth muscles within blood vessel walls, which plays a crucial role in regulating blood flow and blood pressure. When the blood vessels are in a state of vasotonia, they can either be constricted (vasoconstriction) or dilated (vasodilation), which has significant implications for blood pressure regulation and blood flow to different parts of the body. Various factors, including hormonal regulation, neural influences, and local chemical factors, can influence vasotonia. Disturbances in vasotonia can lead to conditions such as hypertension (high blood pressure) or hypotension (low blood pressure). Managing vasotonia is critical for maintaining overall cardiovascular health and preventing related complications. Therefore, providing early and possibly continuous insights into vasotonia is essential to give early and timely understanding to potential diseases of a patient.

As of today, various medical devices have been designed and introduced into the market to measure a caliber of a blood vessel, a typical device is an ultrasonic device or an intravital microscopy. Both devices are known for being cumbersome. Hence, can only be used for measurements at a medical facility. Therefore, not ideal for long-term monitoring, as patients would possibly lose their mobility and flexibility. Further, when visiting a medical facility, the blood vessel caliber will only be monitored for a short period of time reducing the probability to properly diagnose a patient.

Blood vessels can be subdivided into several typical diameters, such as capillaries, arterioles, venules, veins and arteries. Capillaries are the smallest blood vessels in the human body and are involved in the exchange of water, oxygen, carbon dioxide, and many other nutrients and waste substances between blood and the tissues. Their diameter ranges from about 5 to 10 micrometers ($\mu$m) and typically are located very close to the surface of the skin. The depth of capillaries can range approximately 0.2 to 0.5 mm below the skin. Arterioles are small branches of arteries that lead to capillaries. Arterioles have a diameter ranging from about 10 pm to around 100 pm. Venules are small vessels that gather blood from the capillaries and join to form veins. Their diameter can also range from about 10 pm to around 100 pm. Arterioles and venules can be found at varying depths, typically the depth is estimated in range from about 1 to 2 mm but can vary depending on the specific area of the body. Veins and arteries are larger blood vessels and can have significantly larger diameters. Larger blood vessels are usually found deeper in the body. Superficial veins, which are those closest to the skin, can be found at a depth ranging from 2 to 5 mm, and sometimes more, depending on the body part and individual differences. For example, the depth of a large artery can be several centimeters below the skin, depending on the part of the body and the specific area of the body.

In some embodiments, methods and systems are provided suitable for indicating a caliber of a blood vessel below the skin. The system is preferably a wearable that can be attached to the surface of the skin such that the device will not compromise the mobility and flexibility of a patient and can be worn for a longer period of time, thereby increasing the probability to properly diagnose a patient or to monitor the efficacy of a medicament.

In one embodiment, a method for determining blood vessel caliber $D_v$ of blood vessels B below a surface of skin S is described. The method comprises illuminating the skin S with source light 11 having at least a first wavelength $X_a$ and a second wavelength $X_b$. The source light 11 is configured to penetrate into the skin S for interacting with the blood vessels B below the skin S. The second wavelength $X_b$ has a different absorption coefficient $p_a$ for interacting with the blood vessels B than the first wavelength $X_a$. Measuring a first time-dependent signal $S_a$ indicating an intensity $I_a(t)$ variation of light at the first wavelength $X_a$ emanating from the skin S, resulting from the respective source light 11 after having interacted with the blood vessels B below the skin S. Measuring a second time-dependent signal $S_b$ indicating an intensity variation $I_b(t)$ of light at the second wavelength $X_b$ emanating from the skin S, resulting from the respective source light 11 after having interacted with the blood vessels B below the skin S, and determining an indication of the blood vessel caliber $D_v$ based on a combination of the first time-dependent signal $S_a$ and the second time-dependent signal $S_b$. Additional steps or variations may be included to enhance the method's effectiveness in accurately determining blood vessel caliber.

In one embodiment, the first $S_a$ and second $S_b$ time-dependent signals may be measured simultaneously.. In some embodiments, the system 100 comprises multiple detectors 12 or detection elements (e.g. pixels), each configured to measure a distinct wavelengths or ranges of wavelengths. For example, different wavelength filters may be provided to pass the different wavelengths and/or ranges of wavelengths to the different detectors or detection element. Preferably, each distinct wavelength or range of wavelengths is emitted by a respective light source, e.g. different color LEDs. Alternatively, broadband light having multiple wavelengths may be emitted by a respective light source.

Quasi simultaneous measurement of light at different wavelengths can also be achieved by using different light sources each intermittently emitting a different wavelength of light in rapid succession. Preferably, the cycle of emitting light by each light source is relatively short, so that the measurements are well correlated. For example, all flashes of light from each of the different wavelength light sources may occur within 0.1 second, preferably within 0.02 second, most preferably within 0.01 second. The one or more detectors may be gated, e.g. synchronized with the respective light emissions, to determine which wavelength is being measured. To measure a time dependent signal at each wavelength, a series of light flashes may be emitted by each light source. Preferably the flashes are in relatively quick succession, e.g. compared to a physiological cycle time such as heart beat. For example, each light source emits light flashes at a frequency of at least 10 Hz, at least 50 Hz, or at least 100 Hz. The different light sources may emit light flashes at the same frequency, but at different instances of time. For example, the first time-dependent signal $S_a$ and the second time-dependent signal $S_b$ may be measured quasi-simultaneously by emitting flashes of source light having the first wavelength $X_a$ intermittently between emitting flashes of source light having the second wavelength $X_b$. Of course also more than two wavelengths can be used.

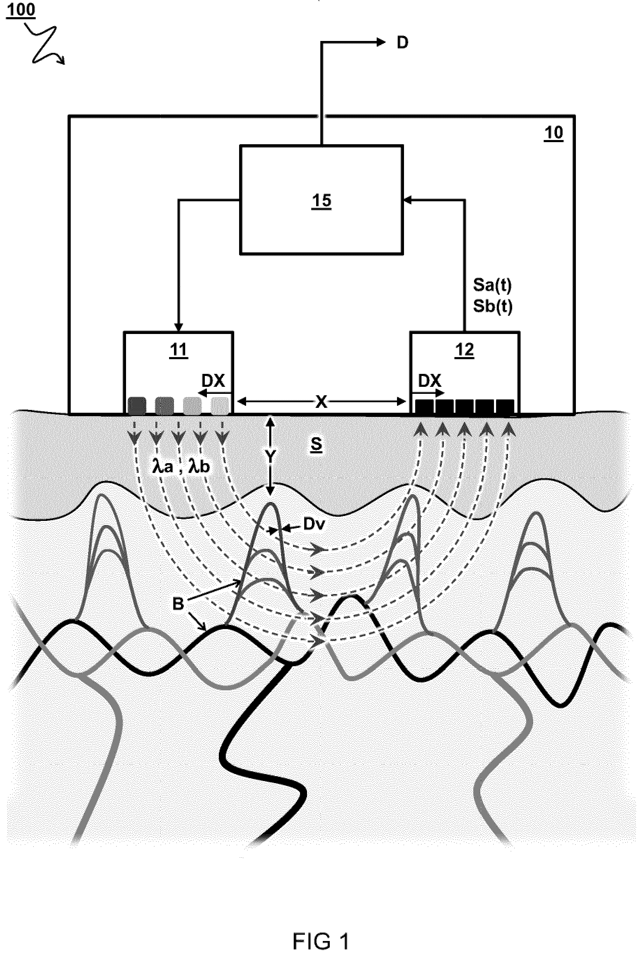

FIG 1 illustrates a system 100 for providing a caliber $D_v$ of a blood vessel B. The device 100 includes a housing 10 that contains one or more light sources 11, one or more detectors 12, and a processor 15. The one or more light sources 11 and detectors 12 are positioned at a distance X from each other, capable of illuminating light at a first $X_a$ and a second wavelength $X_b$ into the skin S. These wavelengths travel through the skin S, interacting with blood vessels B below the skin S and resulting in a first $S_a$ and a second $S_b$ time-dependent signal.

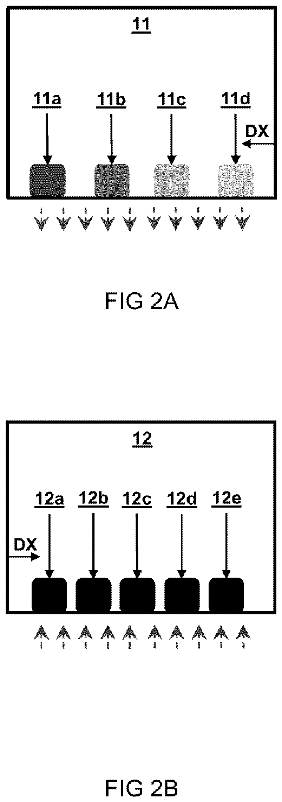

FIG 2A presents an expanded view of a light source 11, comprising multiple light sources 11a - 11d. Each of these light sources 11a - 11d may emit a different wavelength that penetrates the skin S. FIG 2B provides an expanded view of a detector 12. This detector 12 includes multiple detectors 12a - 12e. In one embodiment, different detectors are placed at different distances X+DX from a respective light source. For example, this may be used to measure light at different penetration depths. In another or further embodiment, each detector is capable of measuring a different wavelength after it has interacted with a blood vessel B below the skin S. Alternatively, different detectors arranged at different distances from a respective light source may measure the same wavelength. Alternatively, or in addition to placing different detectors at different distances from the same light source, different light sources can be placed at different distances from the same detector. Also combinations are possible, e.g. having an array of different light sources and an array of different detectors. Various combinations of different light paths and/or different wavelengths can thus be measured.

In some embodiments, the method may include steps for determining a first time-varying component $AC_a$ and a first offset component $DC_a$ of the first time-dependent signal $S_a$, and a second time-varying component $AC_b$ and a second offset component $DC_b$ of the second time-dependent signal $S_b$. The combination of the first $AC_a$ and second $AC_b$ time-varying components may be used to determine an indication of a blood vessel caliber $D_v$. Specifically, an indication of a blood vessel caliber $D_v$ may be based on a combination of the first time-varying component $AC_a$, the first offset component $DC_a$, the second time-varying component $AC_b$, and the second offset component $DC_b$. The first $DC_a$ or second $DC_b$ offset

component may further comprise a fixed offset DCaf, DCbf and/or an actual offset DCaa, DCba.

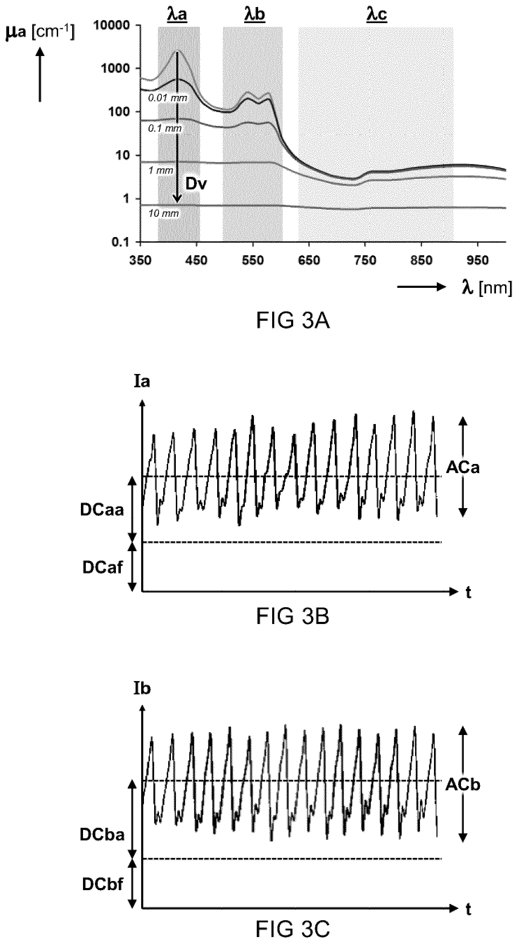

Providing both a time-varying and an offset component may offer more robust information related to the caliber Dv of blood vessel B below the skin S. For instance, an example of a first time-varying component ACa, a first fixed component DCaf, and a first actual offset component DCaa as a function of time is illustrated in FIG 3B. Similarly, FIG 3C shows an example of a second time-varying component ACb, a second fixed component DCbf, and a second actual offset component DCba, again in function of time.

In some embodiments, at least 50% of the measured light at the first wavelength Xa and the second wavelength Xb may have a maximum penetration depth Yp below the surface of the skin S in the range of one to five millimeters, with a preference for between one and three millimeters.

As depicted in FIG 3A, the dependency of the absorption coefficient pa on the blood vessel caliber Dv may be especially pronounced in ranges of Dv up to 0.1 or 1 mm. Arterioles and venules below the skin typically have diameters ranging from about 10 pm to around 100 pm, while veins usually have diameters up to one or two millimeters, with smaller diameters typically found at shallower depths below the skin. Preferably, the light thus particularly measures arterioles and venules predominantly within the dermis, typically at depths of about 1-2 mm, and/or smaller veins and arteries typically at depths of 2 - 5 mm.

In some embodiments, the wavelengths of the time-dependent signals comprise one or more of a wavelength below 500 nm, a wavelength between 500 nm and 650 nm, or a wavelength above 650 nm. Preferably, the first wavelength Xa comprises a wavelength below 500 nm while the second wavelength Xb is selected from a wavelength between 500nm and 650nm, or a wavelength above 650 nm. More preferably, the first wavelength Xa is selected from a wavelength below 500nm and the second wavelength Xb is selected from a wavelength between 500nm and 650nm.

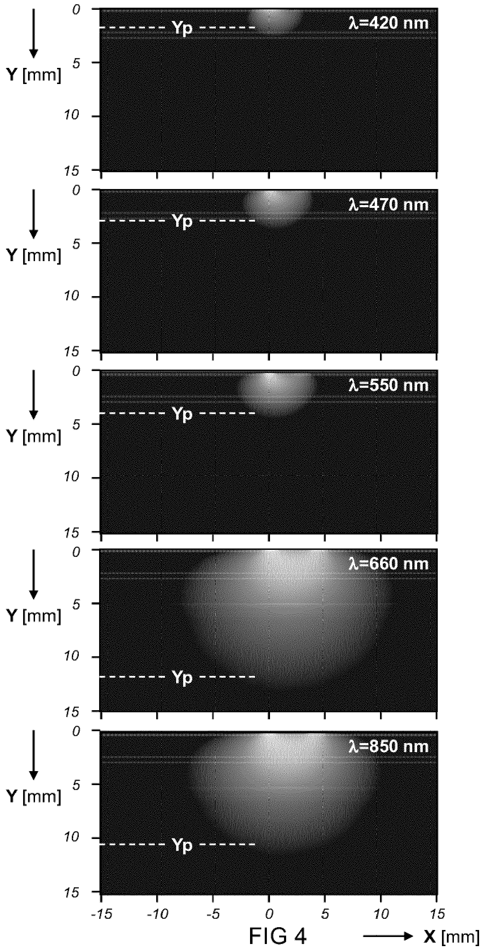

In other or further embodiments, the first wavelength Xa may be 'blue' light selected in a wavelength range between 400 - 450 nm. The second wavelength Xb may be 'green' light selected in a wavelength range between 500 - 600 nm. For example, as illustrated in FIG 4, 'blue' light having a wavelength between 400 - 450 nm may typically have a maximum penetration depth Yp between two and three millimeter; 'green' light having a wavelength between 500 - 600 nm may typically have a maximum penetration depth Yp between three and four millimeter; and 'red' light having a wavelength between 600 - 1100 nm may typically have a maximum penetration depth Yp between ten and fifteen millimeter. So, preferably the measured light includes at least wavelengths Xa and Xb selected from the said ranges of 'blue' and 'green' light. Most preferably, the measured light may also include a third wavelength from said range of 'red' light. As shown in FIG 3A, the 'blue' light and 'green' light have significantly different dependence of the absorption coefficient pa as function of the blood vessel caliber Dv, whereas the 'red' light is relatively unaffected by differences in the vessel caliber (at least up to 0.1 or 1 mm), and may serve as a useful reference or offset.

Alternatively, or in addition to setting one or more suitable wavelengths, the maximum penetration depth Yp may also depend on a separation distance X + DX between a source emitting the light into the skin S, and a sensor 12 measuring the light emitted from the skin S. Typically, the shorter the separation distance X + DX, the more light is measured having a lower penetration depth Yp. Increasing the separation distance X + DX may increase the penetration depth Yp of the measured light. The maximum penetration depth may be limited to a distance and/or depth at which substantially all the light is attenuated, e.g. absorbed below the skin and not reflected.

As illustrated in FIG 4, the lateral spread of the light may typically be similar to the maximum depth of the light. So, to measure light having a desired penetration depth Yp, a distance X + DX between the respective light source and light sensor may be set accordingly. For example, the lateral distance between a respective light source and light sensor is in a range between 0-20 mm, preferably between 2 - 10 mm. In some embodiments, the distance X + DX may be different for different wavelengths of the light, e.g. the larger the penetration depth Yp of the light, the smaller the distance X + DX between the respective light source and sensor. This may be used to compensate the different penetration depths Yp of difference wavelengths. For example, the distance between the respective sensor and source of 'green' light may be smaller than the distance between the respective sensor and source of 'blue' light. For example, the distance between the respective sensor and source of 'red' light may be smaller than the distance between the respective sensor and source of 'blue' light or and/or 'green' light. By tuning the distances between the respective source and sensor, the respective penetration depth may be more similar between the different wavelengths, thus ensuring they measure the same vessels.

In some embodiments, the light having the first wavelength Xa may have a first penetration depth below the skin S and the light having the second wavelength λb may have a second penetration depth below the skin S. The second penetration depth may be larger than the first penetration depth. A first light source 11a emitting the light having the first wavelength may be arranged at a first sensing distance with respect to a first light sensor 12a measuring the light having the first wavelength Xa. A second light source 11b emitting the light having the second wavelength Xb may be arranged at a second sensing distance with respect to a second light sensor 12b measuring the light having the second wavelength Xb. The second sensing distance may be smaller than the first sensing distance. For example, the penetration depth of light below the skin having a particular wavelength, may be expressed using the 1/e "one over e" criterion, where "e" is Euler's number (about 2.72). This is a measure of how deeply light can penetrate into the skin before its intensity decreases to 1/e about 37% of its original intensity on the surface. A change in pathlength and/or penetration depth Yp may possibly result in measuring a different blood vessel calibers Dv. Hence, may affect the accuracy of the measurement. Therefore, variating the sensing distance in function of wavelength may result in substantially similar pathlengths or penetration depths Yp.

In some embodiments, the absorption coefficient pa of the light having the first wavelength Xb depends on the blood vessel caliber Dv according to a first dependence relation. The absorption coefficient pa of the light having the second wavelength Xb depends on the blood vessel caliber Dv according to a second dependence relation. The second dependence relation being different from the first dependence relation.

Preferably, the first and second dependence relations are linearly independent, or at least have some linear independence, e.g. their respective graphs are not only different by a constant scaling factor. For example, the graphs may be nonlinearly correlated and/or nonlinearly dependendent. For example, as shown in FIG 3A, 'blue' light (Xa) may show a greater dependence on the blood vessel caliber Dv than 'green' light (Xb). Furthermore, whereas both 'blue' light and 'green' light may show significant dependence on the blood vessel caliber Dv, this may be much less for the 'red' light (Xc).

Some embodiment comprise illuminating the skin S with source light 11 having a third wavelength Xc. The third wavelength Xc is preferably distinct from the first wavelength Xa and the second wavelength Xb. The third wavelength Xc measuring a third time-dependent signal Sc indicating an intensity variation Ic(t) of the light at the third wavelength Xc emanating from the skin S, resulting from the respective source light 11 after interacted with the blood vessel caliber Dv below the skin S. The indication of the blood vessel caliber Dv may be based on a combination of the first time-dependent signal Sa, the second time-dependent signal Sb, and the third time-dependent signal Sc. In other or further embodiments, the absorption coefficient pa of the light having the third wavelength Xc may be substantially independent of the blood vessel caliber Dv, or at least have a lesser dependence on the blood vessel caliber Dv than the light having the first Xa and second Xb wavelengths. Preferably, the third wavelength Xc is 'red' light selected in a wavelength range between 600 - 1100 nm. The third wavelength may provide for an increased robustness and accuracy.

In a preferred embodiment, the first ACa and/or second ACb time-varying component may be determined by a pigment packaging factor, more preferably a pigment packaging effect PPE. Pigment packaging is a phenomenon related to light transmission through inhomogeneous media, where the absorbing molecules pigments are contained in discrete structures, i.e. spheres, cylinders, instead of homogeneously distributed in the medium. Said pigment packaging effect can also be attributed to hemoglobin in red blood cells and blood vessels. The pigment packaging effect may be calculated by applying the following formula:

$$PPE = \frac{1 - e^{\left(-Dv * \mu_a^{Hb}\right)}}{Dv * \mu_a^{Hb}}$$

where

$$\mu_a^{Hb} = S * \mu_a^{oxy} + (1 - S) * \mu_a^{deoxy}$$

This formula may provide insights into of how a caliber Dv of a blood vessel B can affect the absorption coefficient $\mu_a$ in function of wavelength $\lambda$. The formula comprises an absorption coefficient $\mu_a^{Hb}$ of a blood vessel B. $\mu_a^{Hb}$ may be determined by an absorption coefficient $\mu_a^{oxy}$ being the oxygenated hemoglobin weighted according to an oxygenation S of hemoglobin and an absorption coefficient $\mu_a^{deoxy}$ of deoxygenated hemoglobin weighted by the factor 1-S. The oxygenation S being defined as a fraction of oxyhemoglobin over a total hemoglobin.

In some embodiments, the blood vessel caliber Dv may be determined based on an analytical model which includes an effective absorption coefficient pa of the vessels B at one or more wavelengths. The analytical model may be calculated as function of predetermined absorption coefficients pa of homogeneously distributed oxy- and deoxyhemoglobin at the one or more wavelengths weighted according to an oxygenation S defined as a fraction of oxyhemoglobin over a total hemoglobin. The analytical model further comprises a pigment packaging factor. The pigment packaging factor depending on a caliber Dv of the blood vessel B to determine a pigment packaging effect of absorbing hemoglobin molecules not being homogeneously distributed throughout the blood vessel B but contained in discrete package of the blood vessel B. The blood vessel caliber Dv calculated based on the pigment packaging effect.

For the purpose of clarity and a concise description, features are described herein as part of the same or separate embodiments, however, it will be appreciated that the scope of the invention may include embodiments having combinations of all or some of the features described. For example, while embodiments were shown for determining a caliber of a blood vessel below the skin comprising a pigment packaging effect, also alternative ways may be envisaged by those skilled in the art having the benefit of the present disclosure for achieving a similar function and result. E.g. The wearable may be combined or split up into one or more alternative components. The various elements of the embodiments as discussed and shown offer certain advantages, such as accurate determination of blood vessel calibers, efficacy prediction of medicament, long-term monitoring of blood vessel calibers. Of course, it is to be appreciated that any one of the above embodiments or processes may be combined with one or more other embodiments or processes to provide even further improvements in finding and matching designs and advantages. It is appreciated that this disclosure offers particular advantages to long-term blood vessel monitoring of patients, and in general can be applied for any application wherein where calibers should be determined.

In interpreting the appended claims, it should be understood that the word "comprising" does not exclude the presence of other elements or acts than those listed in a given claim; the word "a" or "an" preceding an element does not exclude the presence of a plurality of such elements; any reference signs in the claims do not limit their scope; several "means" may be represented by the same or different item(s) or implemented structure or function; any of the disclosed devices or portions thereof may be combined together or separated into further portions unless specifically stated otherwise.

**Claims**

1. A method for determining blood vessel caliber (Dv) of blood vessels (B) below a surface of skin (S), the method comprising:

   illuminating the skin (S) with source light having at least a first wavelength (Xa) and a second wavelength ($\lambda$b), wherein the source light is configured to penetrate into the skin (S) for interacting with the blood vessels (B) below the skin (S);
   measuring a first time-dependent signal (Sa) indicating an intensity variation (Ia[t]) of light at the first wavelength (Xa) emanating from the skin (S), resulting from the respective source light after having interacted with the blood vessels below the skin;
   measuring a second time-dependent signal (Sb) indicating an intensity variation (Ib[t]) of light at the second wavelength (Xb) emanating from the skin (S), resulting from the respective source light after having interacted with the blood vessels below the skin; and
   determining an indication of the blood vessel caliber (Dv) based on a combination of the first time-dependent signal (Sa) and the second time-dependent signal (Sb).

2. The method according to claim 1, wherein the first time-dependent signal (Sa) and the second time-dependent signal (Sb) are measured simultaneously by emitting the first wavelength (Xa) and the second wavelength (Xb) at the same time; or quasi-simultaneously by emitting flashes of source light having the first wavelength (Xa) intermittently between emitting flashes of source light having the second wavelength (Xb), wherein the flashes of source light at each wavelength occur at a frequency of at least 10 Hz.

3. The method according to any of the preceding claims, comprising:

   determining a first time-varying component (ACa) and a first offset component (DCa) of the first time-dependent signal (Sa);
   determining a second time-varying component (ACb) and a second offset component (DCb) of the second time-dependent signal;
   wherein the indication of the blood vessel caliber is determined based on a combination of the first time-varying component (ACa), the first offset component (DCa), the second time-varying component (ACb), the second offset component (DCb).

4. The method according to the preceding claim, wherein the blood vessel caliber is based on a ratio of the first time-varying component (ACa) and the first offset component (DCa) and a ratio of the second time-varying component (ACb) and the second offset component (DCb).

5. The method according to any of the preceding claims, wherein at least 50% of the measured light at the first wavelength (Xa) and the second wavelength (Xb) has a maximum penetration depth (Yp) below the surface of the skin in a range between one and five millimeter, preferably between one and three millimeter.

6. The method according to any of the preceding claims, wherein the first wavelength (Xa) is 'blue' light selected in a wavelength range between 400 - 450 nm; and the second wavelength (Xb) is 'green' light selected in a wavelength range between 500 - 600 nm.

7. The method according to any of the preceding claims, wherein the light having the first wavelength (Xa) has a first penetration depth below the skin and the light having the second wavelength (Xb) has a second penetration depth below the skin, wherein the second penetration depth is larger than the first penetration depth, wherein a first light source emitting the light having the first wavelength (Xa) is arranged at a first sensing distance with respect to a first light sensor measuring the light having the first wavelength (Xa), wherein a second light source emitting the light having the second wavelength (Xb) is arranged at a second sensing distance with respect to a second (or the same, first) light sensor measuring the light having the second wavelength (Xb), wherein the second sensing distance is smaller than the first sensing distance.

8. The method according to any of the preceding claims, wherein the absorption coefficient ($\mu$a) of the light having the first wavelength (Xa) depends on the blood vessel caliber (Dv) according to a first dependence relation ($\mu$a[$\lambda$a,Dv]), wherein the absorption coefficient ($\mu$a) of the light having the second wavelength (Xb) depends on the blood vessel caliber (Dv) according to a second dependence relation ($\mu$a[$\lambda$b,Dv]), wherein a graph of the second dependence relation ($\mu$a[$\lambda$b,Dv]) is nonlinearly correlated with a graph of the first dependence relation ($\mu$a[$\lambda$a,Dv]).

9. The method according to any of the preceding claims, wherein the method comprises illuminating the skin (S) with source light having a third wavelength (Xc), distinct from the first wavelength (Xa) and the second wavelength (Xb); and measuring a third time-dependent signal indicating an intensity variation of light at the third wavelength emanating from the skin (S), resulting from the respective source light after having interacted with the blood vessels below the skin, wherein the indication of the blood vessel caliber (Dv) is based on a combination of the first time-dependent signal (Sa), the second time-dependent signal (Sb), and the third time-dependent signal (Sc).

10. The method according to the preceding claim, wherein the absorption coefficient ($\mu$a) of the light having the third wavelength (Xc) is substantially independent of the blood vessel caliber (Dv), or at least having a lesser dependence on the blood vessel caliber (Dv) than the light having the first and second wavelengths.

11. The method according to any the two preceding claims, wherein the third wavelength (Xc) is 'red' light selected in a wavelength range between 600 - 1100 nm

12. The method according to any of the preceding claims, wherein the blood vessel caliber (Dv) is determined based on an analytical model which includes an effective absorption coefficient of the vessels at the one or more wavelengths calculated as function of

a predetermined absorption coefficients of homogenously distributed oxy- and deoxyhemoglobin at the one or more wavelengths weighted according to an oxygenation (S) defined as a fraction of oxyhemoglobin over a total hemoglobin; and
a pigment packaging factor depending on a caliber of the blood vessel to determine a pigment packaging effect of absorbing hemoglobin molecules not being homogeneously distributed throughout the blood vessel but contained in discrete package of the blood vessel;
wherein the blood vessel caliber is calculated based on the pigment packaging effect.

13. A non-transitory computer-readable medium storing instructions that, when executed by one or more processors, cause a device to perform the method according to any of the preceding claims.

14. A system for determining blood vessel caliber (Dv) of blood vessels (B) below a surface of skin (S), the system comprising:

one or more light sources configured to illuminate the skin (S) with source light having at least a first wavelength (Xa) and a second wavelength ($\lambda$b), wherein the source light is configured to penetrate into the skin (S) for interacting with the blood vessels (B) below the skin (S), wherein the second wavelength ($\lambda$b) has a different absorption coefficient ($\mu$a) for interacting with the blood vessels than the first wavelength ($\lambda$a);
one or more light sensors configured to measure a first time-dependent signal (Sa) indicating an intensity variation (Ia[t]) of light at the first wavelength ($\lambda$a) emanating from the skin (S), resulting from the respective source light after having interacted with the blood vessels below the skin, and measure a second time-dependent signal

(Sb) indicating an intensity variation (Ib[t]) of light at the second wavelength ($\lambda$b) emanating from the skin (S), resulting from the respective source light after having interacted with the blood vessels below the skin; and
a processor configured to determine an indication of the blood vessel caliber (Dv) based on a combination of the first time-dependent signal (Sa) and the second time-dependent signal (Sb).

15. The system according to the preceding claim, wherein the processor is configured to output the indication of the blood vessel caliber (Dv) onto a display or other output device.

FIG 1

FIG 2A

FIG 2B

FIG 3A

FIG 3B

FIG 3C

13

FIG 4

Europäisches
Patentamt

European
Patent Office

Office européen
des brevets

# EUROPEAN SEARCH REPORT

**Application Number**

EP 24 15 2650

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2015/216425 A1 (GLADSHTEIN REUVEN [IL] ET AL) 6 August 2015 (2015-08-06) | 1,2,5, 7-9, 13-15 | INV. A61B5/00 A61B5/107 |
| Y | * paragraph [0010]; claims 115,122; figure 1 * | 3,4,10, 11 | |
| A | * paragraph [0071] * <br> * paragraph [0144] – paragraph [0153] * | 6,12 | |
| Y | US 2018/042522 A1 (SUBRAMANIAN HARIHARAN [US] ET AL) 15 February 2018 (2018-02-15) <br> * paragraph [0027] * <br> * paragraph [0034] – paragraph [0035] * | 3,4,10, 11 | |
| A | WO 2014/184447 A1 (PULSEON OY [FI]) 20 November 2014 (2014-11-20) <br> * page 16, line 33 – line 35; figure 1A * <br> * page 17, line 1 – line 9 * | 7 | |
| A | US 2022/296115 A1 (CHOI JIN WOO [KR] ET AL) 22 September 2022 (2022-09-22) <br> * paragraph [0018] – paragraph [0020] * <br> * paragraph [0055] – paragraph [0061] * <br> * paragraph [0116] * | 1-4,8,9, 11,13-15 | TECHNICAL FIELDS SEARCHED (IPC) <br><br> A61B |
| A | US 2020/330011 A1 (HONORE FRANCIS [US] ET AL) 22 October 2020 (2020-10-22) <br> * paragraph [0007] – paragraph [0011] * <br> * paragraph [0057] – paragraph [0062] * <br> * paragraph [0096] * | 1,2,7, 13-15 | |
| A | EP 3 745 957 B1 (STICHTING VU [NL]) 27 April 2022 (2022-04-27) <br> * paragraph [0017]; figures 1A,1B,13 * | 6,8,12 | |

The present search report has been drawn up for all claims

| Place of search | Date of completion of the search | Examiner |
|---|---|---|
| The Hague | 27 March 2024 | Costa Angeli, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.                    EP 24 15 2650

This annex lists the patent family members relating to the patent documents cited in  the above-mentioned European search report.
The members are as contained in the European Patent Office EDP file on
The European Patent Office is in no way liable for these particulars which are merely given for the purpose of information.

27-03-2024

| Patent document cited in search report | | | Publication date | Patent family member(s) | | | Publication date |
|---|---|---|---|---|---|---|---|
| US 2015216425 | A1 | | 06-08-2015 | GB | 2519909 | A | 06-05-2015 |
| | | | | US | 2015216425 | A1 | 06-08-2015 |
| | | | | WO | 2014027293 | A3 | 10-04-2014 |
| US 2018042522 | A1 | | 15-02-2018 | EP | 3258840 | A1 | 27-12-2017 |
| | | | | ES | 2892526 | T3 | 04-02-2022 |
| | | | | JP | 6763868 | B2 | 30-09-2020 |
| | | | | JP | 2018507047 | A | 15-03-2018 |
| | | | | US | 2018042522 | A1 | 15-02-2018 |
| | | | | WO | 2016134327 | A1 | 25-08-2016 |
| WO 2014184447 | A1 | | 20-11-2014 | CN | 105208924 | A | 30-12-2015 |
| | | | | EP | 2996553 | A1 | 23-03-2016 |
| | | | | ES | 2830674 | T3 | 04-06-2021 |
| | | | | FI | 126338 | B | 14-10-2016 |
| | | | | JP | 6501760 | B2 | 17-04-2019 |
| | | | | JP | 2016521190 | A | 21-07-2016 |
| | | | | KR | 20160008581 | A | 22-01-2016 |
| | | | | US | 2016081567 | A1 | 24-03-2016 |
| | | | | WO | 2014184447 | A1 | 20-11-2014 |
| US 2022296115 | A1 | | 22-09-2022 | CN | 115105039 | A | 27-09-2022 |
| | | | | EP | 4059418 | A1 | 21-09-2022 |
| | | | | KR | 20220130905 | A | 27-09-2022 |
| | | | | US | 2022296115 | A1 | 22-09-2022 |
| US 2020330011 | A1 | | 22-10-2020 | AU | 2018244596 | A1 | 17-10-2019 |
| | | | | CA | 3057979 | A1 | 04-10-2018 |
| | | | | CN | 110868920 | A | 06-03-2020 |
| | | | | EP | 3600020 | A1 | 05-02-2020 |
| | | | | JP | 2020515367 | A | 28-05-2020 |
| | | | | JP | 2023138651 | A | 02-10-2023 |
| | | | | US | 2020330011 | A1 | 22-10-2020 |
| | | | | US | 2021212616 | A1 | 15-07-2021 |
| | | | | WO | 2018183558 | A1 | 04-10-2018 |
| EP 3745957 | B1 | | 27-04-2022 | EP | 3745957 | A1 | 09-12-2020 |
| | | | | ES | 2924301 | T3 | 05-10-2022 |
| | | | | WO | 2019147135 | A1 | 01-08-2019 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2002042558 A1
- EP 3745957 A1